# EUROPÄISCHE PATENTANMELDUNG

(11) **EP 1 844 807 A1**
(43) Veröffentlichungstag der Anmeldung: **17.10.2007**
(21) Anmeldenummer: 06405161.8
(22) Anmeldetag: 13.04.2006
(51) Int. Cl.: A61M 15/00

(54) **Medikamentenmagazin, sowie Vorrichtung und Verfahren zum Öffnen desselben; Mehrdosispulverinhalator**

(71) Anmelder: Boehringer Ingelheim Pharma GmbH & Co. KG, 55216 Ingelheim am Rhein (DE)
(72) Erfinder: Wachtel, Herbert, 55218 Ingelheim am Rhein (DE); Geser, Johannes, 55218 Ingelheim am Rhein (DE); Metzger, Burkhard, 55218 Ingelheim am Rhein (DE); Spallek, Michael, 55218 Ingelheim am Rhein (DE); Krueger, Michael, 55218 Ingelheim am Rhein (DE); Kunze, Hubert, 44227 Dortmund (DE); Moser, Achim, 65843 Sulzbach (DE); Mock, Elmar, 2013 Colombier (CH); Lanci, Antonino, 3006 Bern (CH); Klopfenstein, André, 2520 La Neuveville (CH)
(74) Vertreter: Frei Patent Attorneys

(57) **Zusammenfassung**

Die Erfindung betrifft ein Medikamentenmagazin mit einer Mehrzahl von Medikamentendosen, wobei das Magazin aus einer einzigen Folienbahn gebildet ist, in welcher Taschen (2) zur Aufnahme eines Medikaments geformt sind. Die Folienbahn weist für den Transport der Bahn auf mindestens einer Seite Öffnungen (4) auf für den Eingriff von Transportstacheln. Die Folienbahn weist im Bereich der Taschen eine gewisse Breite auf, welche kleiner ist als die Breite der Folie in übrigen Bereichen, und dass die Öffnungen in diesem breiteren Bereich der Folienbahn angeordnet sind. Die Erfindung betrifft auch ein Verfahren und eine Vorrichtung zum Öffnen eines solchen Medikamentenmagazins, wobei die Vorrichtung bevorzugt als Segmentrad ausgebildet ist.

## Beschreibung

Die Erfindung liegt auf dem Gebiet von Medikamentenmagazinen, insbesondere von Medikamentenmagazinen, in welchen in einer einzelnen Folienbahn Taschen zur Aufnahme eines Medikaments gebildet sind, gemäss dem Oberbegriff des unabhängigen Patentanspruchs. Ebenfalls Gegenstand der Erfindung sind eine Vorrichtung und ein Verfahren zum Öffnen des Medikamentenmagazins, sowie ein Mehrdosispulverinhalator.

Aus dem Dokument US 2005/0172962 ist ein Folienblister bekannt, in welchem ein einzelnes Folienband zu einzelnen Medikamententaschen gefaltet ist. Das Band weist einseitig entlang seiner Länge Öffnungen auf. In diese Öffnungen greift ein Zahnrad ein, welches den Blister blockiert, sobald eine Medikamententasche ein Piezoelement erreicht hat. Eine Aufwickelrolle auf der gegenüberliegenden Seite des Piezoelements wickelt das Folienband auf. Die Rolle zieht dabei solange an der Folie bis die entsprechende Medikamententasche geöffnet ist und das darin befindliche Medikament mit Hilfe des Piezoelements aus der Folie geschüttelt werden kann. Dieser Blister und Öffnungsmechanismus weist mehrere Nachteile auf. Zum einen muss das Blisterband inklusive Tasche über das Zahnrad rollen. Dazu müssen Öffnungen in den Taschen und des übrigens Bandes genau übereinstimmen um nicht vom Zahnrad 'gehoben' zu werden. Auch lässt dies ein zuverlässiges Einrasten des Rades im Blister nach dem Durchgang der Tasche fraglich erscheinen. Zudem müssen die einzelnen Taschen um mehr als eine Taschenlänge voneinander beabstandet sein, um nicht auf dem Zahnrad aufeinander zu liegen. Dies begrenzt jedoch eine gewünschte hohe Dichte an hintereinander angeordneten Taschen. Des weiteren erfordert der beschriebene Öffnungsmechanismus viel Platz aufgrund der weit auseinander liegenden Räder.

Es ist deshalb Aufgabe der Erfindung, den bekannten Einfolien-Faltblister zu verbessern und genannte Nachteile zu beheben, so dass vielseitigerer Möglichkeiten in Bezug auf die Handhabung des Folienblisters möglich sind.

Die Erfindung wird durch das Medikamentenmagazin und die Vorrichtung bzw. das Verfahren gelöst, wie sie in den unabhängigen Ansprüchen definiert sind.

Bevorzugte Ausführungsformen der Erfindung sind in den Unteransprüchen beschrieben.

Das erfindungsgemässe Medikamentenmagazin weist eine Mehrzahl von Medikamentendosen auf, wobei das Magazin aus einer einzigen Folienbahn gebildet ist, in welcher Taschen zur Aufnahme der Medikamentendosen geformt sind. Die Folienbahn weist für den Transport der Bahn auf mindestens einer Seite Öffnungen auf für den Eingriff von Transportstacheln. Dabei weist die Folienbahn im Bereich der Taschen eine gewisse Breite auf, welche kleiner ist als die Breite der Folie in übrigen Bereichen, wobei die Öffnungen in diesem breiteren Bereich der Folienbahn angeordnet sind.

Dies bietet den Vorteil, dass der Folienblister seitlich geführt werden kann, im wesentlichen unabhängig von der Position, Lage und Masse der einzelnen Taschen. Die Taschen können Platz sparend, z. B. schuppenförmig, übereinander liegen, ohne den Transport des Bandes zu beeinflussen. Auch können Transport- und Öffnungsmittel unmittelbar aneinander anschliessen oder in einem einzigen Element kombiniert werden. Die Taschen können dabei direkt neben einem Transport- und/oder Öffnungsmittel, beispielsweise in einem Zwischenraum zwischen zwei parallel angeordneten Rädern, angeordnet werden. Ein Folienblister und eine Vorrichtung, in welche ein solcher Folienblister eingebracht wird, können damit sehr Platz sparend gestaltet werden.

Eine Folienbahn weist nach ihrer Verarbeitung zu einem Medikamentenmagazin mindestens eine Tasche auf, in welcher ein Medikament, beispielsweise ein Pulver, eingebracht ist. Vor bzw. hinter der mindestens einen Tasche, bei mehreren Taschen zwischen diesen, ist das Folienband breiter und weist die Öffnungen für einen Transport des Blisters, vorzugsweise auch für ein Öffnen des Blisters auf. Der die Öffnungen aufweisende breitere Bereich des Blisters bildet einen Rand entlang des Magazins, welcher vorzugsweise im wesentlichen so breit ist wie die Öffnungen. Da die Öffnungen jedoch lediglich zwischen den Taschen angeordnet sind, ist die Breite der Öffnungen unkritisch: Es wird keine Medikamentenkammer durch das Anbringen von Öffnungen beschädigt oder die Grösse einer solchen Kammer durch die Grösse der Öffnungen beeinträchtigt.
Vorzugsweise befinden sich in beiden Ränder der Folienbahn Öffnungen. Dies erlaubt ein sehr kontrolliertes, insbesondere symmetrisches, Transportieren und Öffnen des Magazins bzw. einer Tasche.
Die Anordnung und Gestaltung der Öffnungen kann zum Transportieren, Arretieren und Öffnen eines Blisters verwendet werden. Es ist aber auch möglich diese für weitere Funktionen, z. B. eine Indexierung und/oder Kontrolle zu verwenden. Über unterschiedliche Grössen, z. B. Längen, oder Abständen von Öffnungen kann beispielsweise zwischen einzelnen Taschen unterschieden werden. Es ist auch möglich darüber eine Anzeige von verbrauchten bzw. noch verwendbaren Medikamentendosen zu steuern.

Die Medikamententaschen können symmetrisch in der Folienbahn eingebracht sein. Es ist aber auch möglich eine Seite der Tasche mit einer Vertiefung zu versehen und die Abdeckung dann als im wesentlich ebene Fläche zu gestalten, oder mit der Folie vorerst eine Schlaufe zu formen und diese dann durch geeignete Versiegelungen vor bzw. nach dem Füllen zu verschliessen.

Eine Vorrichtung zum Öffnen eines Medikamentenmagazins weist mindestens einen bewegbaren Stachel zum Eingriff in eine dafür vorgesehene Öffnung des Magazins auf. Zudem weist sie Mittel für einen Transportschub eines Magazins und Mittel für einen Öffnungsschub eines Magazins zum Öffnen einer in einer Folienbahn eingebrachten Tasche auf. Dabei ist der mindestens eine Stachel derart angeordnet, dass er in Bezug auf eine Transportrichtung seitlich und beabstandet zur Tasche in die Folienbahn eingreift. Ein solcher Transportstachel ist bevorzugt ein Zahn eines Zahnrades, wobei in einer bevorzugten Ausführungsform mindestens zwei Stacheln parallel zueinander und um mindestens eine Medikamententaschenbreite beabstandet voneinander angeordnet sind, derart, dass eine Tasche zwischen den Stacheln bzw. einem die Stacheln aufweisenden Transport- und/oder Öffnungsmittel im wesentlichen frei bewegbar ist.

In der Vorrichtung zum Öffnen eines Medikamentenmagazins sind Transport- und Öffnungsmittel vorzugsweise in einem einzigen Mittel kombiniert, welches Mittel bevorzugt als diskontinuierliches Stachelrad, z. B. ein Segmentrad, ausgebildet ist.

Beim Transportieren und Öffnen eines Medikamentenmagazins greift mindestens ein Stachel in eine dafür vorgesehene Öffnung des vorzugsweise eine Mehrzahl von Medikamententaschen aufweisenden Medikamentenmagazins ein und bringt durch eine Transportbewegung das Magazin in eine Öffnungsposition. Der Teil des Magazins, welches sich vor einer Medikamententasche im Bereich einer Öffnungsposition befindet wird festgehalten und der Teil des Magazins, welches sich nach dieser Medikamententasche befindet wird mittels dem mindestes einen Stachel weitertransportiert. Dadurch wird die Medikamententasche aufgezogen, in dem die aneinander befestigten Folienbereiche voneinander gelöst werden, und das in der Medikamententasche befindliche Medikament wird, beispielsweise zur Inhalation, frei gegeben.

Eine Medikamententasche wird vorzugsweise durch mindestens zwei in das Medikamentenmagazin eingreifende und sich relativ voneinander wegbewegende Stacheln geöffnet. Sind die Stacheln Teil eines Segmentrades, so gehören die Stacheln zu unterschiedlichen Segmenten des Rades und eine Medikamententasche wird durch ein Auseinander bewegen bzw. Öffnen dieser Segmente geöffnet. Eine Medikamentenkammer ist dabei zwischen den zwei Segmenten des Segmentrades angeordnet.

Das erfindungsgemässe Medikamentenmagazin und die Öffnungsvorrichtung werden typischerweise in einer Medikamentenausgabevorrichtung, vorzugsweise einem Inhalator, wie beispielsweise einem Mehrdosispulverinhalator, verwendet. Die Dosenanzahl liegt dabei vorzugsweise in einem Bereich von 1 bis 100 oder bis 200 Einzeldosen, bevorzugt in einem Bereich von 1-60, beispielsweise zwischen 7-180 oder 14-150, z. B. 30-120, 45-100, 30, 90, 60, 120. Für Inhalationsgeräte liegt eine Maximalanzahl von Einzeldosen aus Handlichkeits- und Therapie-Gründen vorzugsweise bei 60.

Ein erfindungsgemässer Mehrdosispulverinhalator weist somit ein erfindungsgemässes Medikamentenmagazin und/oder eine erfindungsgemässe Vorrichtung zum Öffnen eines solchen Magazins auf.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Besonders bevorzugt sind in diesem Zusammenhang Arzneimittel, die ausgewählt sind aus der Gruppe bestehend aus Anticholinergika, Betamimetika, Steroiden, Phosphodiesterase N-inhibitoren, LTD4-Antagonisten und EGFR-Kinase-Hemmer, Antiallergika, Derivate von Mutterkornalkaloiden, Triptane, CGRP-Antagonisten, Phosphodiesterase-V-Inhibitoren, sowie Kombinationen aus solchen Wirkstoffen, z.B. Betamimetika plus Anticholinergika oder Betamimetica plus Antiallergika. Bevorzugt werden Anticholinergika-haltige Wirkstoffe eingesetzt, als Monopräparate oder in Form von Kombinationspräparaten.

Im einzelnen seien als Beispiele für die wirksamen Bestandteile oder deren Salze genannt:

Zur Anwendung gelangende Anticholinergika sind bevorzugt ausgewählt aus der Gruppe bestehend aus Tiotropiumbromid, Oxitropiumbromid, Flutropiumbromid, Ipratropiumbromid, Glycopyrroniumsalze, Trospiumchlorid, Tolterodin, 2,2-Diphenylpropionsäuretropenolester-methobromid, 2,2-Diphenylpropionsäurescopinester-methobromid, 2-Fluor-2,2-Diphenylessigsäurescopinester-methobromid, 2-Fluor-2,2-Diphenylessigsäuretropenolester-methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäurescopinester-Methobromid, 9-Hydroxy-fluoren-9-carbonsäuretropenolester -Methobromid, 9-Fluor-fluoren-9-carbonsäuretropenolester -Methobromid, 9-Hydroxy-fluoren-9-carbonsäurescopinester -Methobromid, 9-Fluor-fluoren-9-carbonsäurescopinester Methobromid, 9-Methyl-fluoren-9-carbonsäuretropenolester Methobromid, 9-Methyl-fluoren-9-carbonsäurescopinester Methobromid, Benzilsäurecyclopropyltropinester-Methobromid, 2,2-Diphenylpropionsäurecyclopropyltropinester -Methobromid, 9-Hydroxy-xanthen-9-carbonsäurecyclopropyltropinester-Methobromid, 9-Methyl-fluoren-9-carbonsäurecyclopropyltropinester-Methobromid, 9-Methyl-xanthen-9-carbonsäurecyclopropyltropinester-Methobromid, 9-Hydroxy-fluoren-9-carbonsäurecyclopropyltropinester-Methobromid, 4,4'-Difluorbenzilsäuremethylestercyclopropyltropinester -Methobromid, 9-Hydroxy-xanthen-9-carbonsäuretropenolester -Methobromid, 9-Hydroxy-xanthen-9-carbonsäurescopinester Methobromid, 9-Methyl-xanthen-9-carbonsäuretropenolester -Methobromid, 9-Methyl-xanthen-9-carbonsäurescopinester -Methobromid, 9-Ethyl-xanthen-9-carbonsäuretropenolester Methobromid, 9-Difluormethyl-xanthen-9-carbonsäuretropenolester -Methobromid und 9-Hydroxymethyl-xanthen-9-carbonsäurescopinester -Methobromid, gegebenenfalls in Form ihrer Racemate, Enantiomere oder Diastereomere und gegebenenfalls in Form ihrer Solvate und/oder Hydrate.

Zur Anwendung gelangende Betamimetika sind bevorzugt ausgewählt aus der Gruppe bestehend aus Albuterol, Bambuterol, Bitolterol, Broxaterol, Carbuterol, Clenbuterol, Fenoterol, Formoterol, Hexoprenaline, Ibuterol, Indacaterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenot, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethylphenyl)-ethylamino]-hexyloxy}-butyl)-benzolsulfonamid, 5-[2-(5,6-Diethyl-indan-2-ylamino)-1-hydroxy-ethyl]-8-hydroxy-1*H*-quinolin-2-on, 4-hydroxy-7-[2-{[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol , 1-[2H-5-hydroxy-3-oxo-4H-1,4-benzoxazin-8-yl]-2-[3-(4-N,N-dimethylaminophenyl)-2-methyl-2-propylamino]ethanol , 1-[2H-5-hydroxy-3-oxo-4H-1,4-benzoxazin-8-yl]-2-[3-(4-methoxyphenyl)-2-methyl-2-propylamino]ethanol , 1-[2H-5-hydroxy-3-oxo-4H-1,4-benzoxazin-8-yl]-2-[3-(4-n-butyloxyphenyl)-2-methyl-2-propylamino]ethanol , 1-[2H-5-hydroxy-3-oxo-4H-1,4-benzoxazin-8-yl]-2- {4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol und 1-(4-ethoxycarbonylamino-3-cyano-5-fluorophenyl)-2-(tert.-butylamino)ethanol, gegebenenfalls in Form ihrer Racemate, Enantiomere oder Diastereomere und gegebenenfalls in Form ihrer pharmakologisch verträglichen Säureadditionssalze, Solvate und/oder Hydrate.

Zur Anwendung gelangende Steroide sind bevorzugt ausgewählt aus der Gruppe bestehend aus Prednisolon, Prednison, Butixocortpropionat, RPR-106541, Flunisolid, Beclomethason, Triamcinolon, Budesonid, Fluticason, Mometason, Ciclesonid, Rofleponid, ST-126, Dexamethason, 6α,9α-Difluoro-17α-[(2-furanylcarbonyl)oxy]-11β-hydroxy-16α-methyl-3-oxo-androsta-1,4-dien-17β-carbothionsäure (S)-fluoromethylester, 6α,9α-Difluoro-11β-hydroxy-16α-methyl-3-oxo-17α-propionyloxy-androsta-1,4-dien-17β-carbothionsäure(S)-(2-oxo-tetrahydro-furan-3S-yl)ester und Etiprednol-dichloroacetat (BNP-166), gegebenenfalls in Form ihrer Racemate, Enantiomere oder Diastereomere und gegebenenfalls in Form ihrer Salze und Derivate, ihrer Solvate und/oder Hydrate.

Zur Anwendung gelangende PDE IV-Inhibitoren sind bevorzugt ausgewählt aus der Gruppe bestehend aus Enprofyllin, Theophyllin, Roflumilast, Ariflo (Cilomilast), CP-325,366, BY343, D-4396 (Sch-351591), AWD-12-281 (GW-842470), N-(3,5-Dichloro-1-oxo-pyridin-4-yl)-4-difluoromethoxy-3-cyclopropylmethoxybenzamid, NCS-613, Pumafentine, (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, CDP840, Bay-198004, D-4418, PD-168787, T-440, T-2585, Arofyllin, Atizoram, V-11294A, C1-1018, CDC-801, CDC-3052, D-22888, YM-58997, Z-15370, 9-Cyclopentyl-5,6-dihydro-7-ethyl-3-(2-thienyl)-9*H*-pyrazolo[3,4-c]-1,2,4-triazolo[4,3-a]pyridin und 9-Cyclopentyl-5,6-dihydro-7-ethyl-3-(*tert*-butyl)-9*H*-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Zur Anwendung gelangende LTD4-Antagonisten sind bevorzugt ausgewählt aus der Gruppe bestehend aus Montelukast, 1-(((R)-(3-(2-(6,7-Difluoro-2-quinolinyl)ethenyl)phenyl)-3-(2-(2-hydroxy-2-propyl)phenyl)thio)methylcyclo-propan-essigsäure, 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclo-propanessigsäure, Pranlukast, Zafirlukast, [2-[[2-(4-tert-Butyl-2-thiazolyl)-5-benzofuranyl]oxymethyl]phenyl]essigsäure, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Zur Anwendung gelangende EGFR-Kinase-Hemmer sind bevorzugt ausgewählt aus der Gruppe bestehend aus Cetuximab, Trastuzumab, ABX-EGF, Mab ICR-62, 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluorphenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluorphenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenylethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluorphenyl)amino]-6-{1-[(morpholin-4-yl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin, 4-[(3-Chlor-4-fluorphenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methylpiperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluorphenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluorphenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die Verbindungen gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als Antiallergika: Dinatriumcromoglicat, Nedocromil.

Als Derivate der Mutterkornalkaloide: Dihydroergotamin, Ergotamin.

Für die Inhalation kommen Arzneimittel, Arzneimittelformulierungen und - mischungen mit den o.g. Wirkstoffen in Betracht, sowie deren Salze, Ester sowie die Kombination dieser Wirkstoffe, Salze und Ester.

Im Folgenden wird die Erfindung anhand von Beispielen und Figuren weiter erläutert, es zeigen:
- Fig. 1: ein Einfolien-Faltblister
- Fig. 2: eine Abfolge eines Öffnungsvorganges eines Einfolien-Faltblisters
- Fig. 3: eine weitere Ausführungsform eines Einfolien-Faltblisters
- Fig. 4: Ein Transport- und Öffnungsmechanismus in der Form eines Segmentrades
- Fig. 5a-d: Verschiedene Öffnungspositionen eines Faltblisters

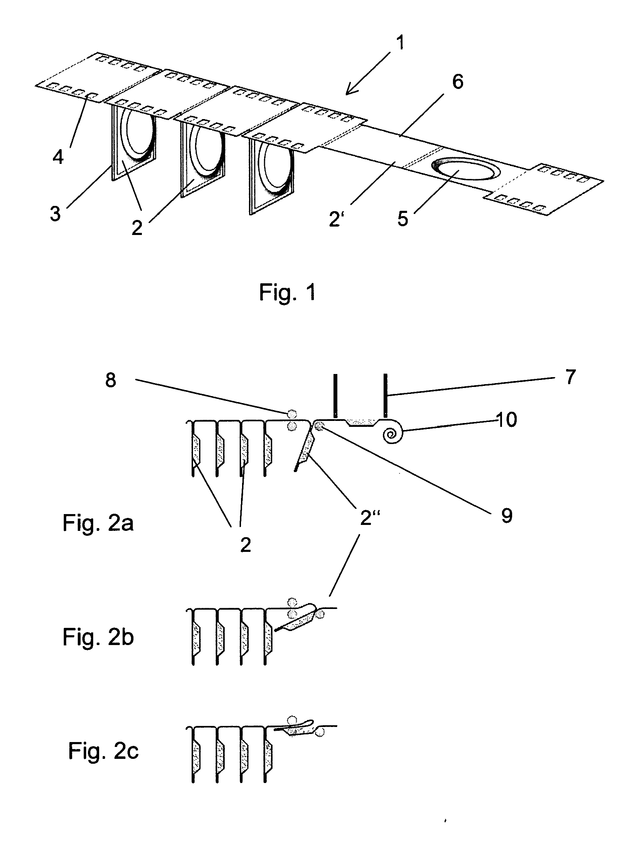

**Figur 1** zeigt einen Faltblister 1, welcher aus einem einzelnen Folienband hergestellt ist. Im Folienband sind in regelmässigen Abständen Taschen 2 geformt, welche vorzugsweise ein pulverförmiges Medikament beinhalten. Um ein Austreten von Medikament aus den Taschen zu verhindern, sind diese an ihren Rändern versiegelt (Siegelung 3). Eine geöffnete Medikamententasche 2' weist auf der einen Seite der Tasche eine napfförmige Vertiefung 5 im Folienband auf, während die andere Seite eine im wesentlichen ebene Abdeckung ist. Diese Art der Foliengestaltung erlaubt ein Füllen der Taschen, bei dem im wesentlichen jeweils nur die Abdeckung bewegt, d. h. aufgebracht und entfernt, werden muss und z. B. ein Pulver, auch beim oder nach einem Öffnen einer Tasche, ohne Gefahr zu laufen herauszufallen, in der Vertiefung liegt.

Die Taschen sind parallel zueinander und senkrecht zur Bandlängsachse angeordnet, wobei eine Transportrichtung des Blisters typischerweise auch entlang der Bandlängsachse verläuft. Dieses hintereinander Anordnen der Taschen, erlaubt eine sehr hohe Packungsdichte.

Der Bereich 6 des Folienbandes, welcher eine Tasche bildet, weist eine geringere Breite auf, als das übrige Folienband. In den breiteren, gegenüber den Taschen seitlich vorstehenden Bereichen des Folienbandes sind Öffnungen 4 eingebracht. Diese Öffnungen dienen zum Eingreifen von Transport- oder Öffnungsstacheln eines entsprechenden Transport- und/ oder Öffnungsmittels, z.B. eines Rades.

Die Öffnungen befinden sich nur zwischen den einzelnen Taschen. Die Öffnungen sind derart angeordnet, dass sie nach einer Taschenbildung eine im wesentlichen kontinuierliche Reihe von Öffnungen bilden. Es ist jedoch möglich, z. B. durch unterschiedlich grosse Abstände, durch unterschiedlich grosse Öffnungen, durch Aussparungen etc. die einzelnen Taschen voneinander zu unterscheiden (Indizierung). Beispielsweise können mehrere gleich grosse Öffnungen für einen Transport des Blisters vorgesehen sein, jedoch nur eine einzelne grössere Öffnung pro Medikamententasche. Ein beispielsweise für eine Arretierung vorgesehener breiter Stachel kann entsprechend nur in diese grössere Öffnung eingreifen und damit ein Arretieren auslösen.

Aufgrund der speziellen Gestaltung des Blisters ist es möglich eine einzige seitliche Öffnung pro Tasche vorzusehen und benachbarte Taschen bis auf den Abstand für diese seitliche Öffnung direkt aneinander anschliessend anzuordnen.

Dadurch, dass die Taschenbereiche schmaler sind, kann der Folienblister seitlich geführt werden. Die gesamte Handhabung des Folienblisters ist im wesentlichen unabhängig von der Position und Lage der einzelnen Taschen. Insbesondere ein oder mehrere Transportstacheln eines oder mehrerer Transporträder können an beliebiger Lage/zu beliebigem Zeitpunkt in den Blister seitlich eingreifen. Es muss keine genaue Position zwischen Taschen abgepasst werden. Auch ein Passieren einer Tasche über ein Zahnrad fällt weg. Die Taschen können Platz sparend, schuppenförmig übereinanderliegen, ohne den Transport des Bandes zu beeinflussen. Von besonderem Vorteil ist diese Unabhängigkeit auch für eine Übergabe, beispielsweise von einem Transport- auf ein Öffnungsrad: Beide können im wesentlichen direkt aneinander anschliessen. Die Taschen befinden sich dabei neben einem Rad oder in einem Zwischenraum zwischen zwei parallel angeordneten Rädern, der in der Regel sowieso aufgrund des Durchmesser eines solchen Rades vorhanden ist. Transport- und Öffnungsmechanismen können so nahe beieinander angeordnet werden. Dies bietet auch Vorteile in Bezug auf eine Medikamentenausgabevorrichtung, in welche ein solcher Folienblister eingebracht wird. Diese kann im wesentlichen unabhängig von einer Taschentiefe bzw. einem Taschenabstand gestaltet sein, insbesondere wenn ein Öffnungsmechanismus durch die Anordnung und/oder Gestaltung der seitlichen Öffnungen ausgelöst wird.

In den **Figuren 2a-c** ist ein Ablauf eines Öffnungsvorganges eines Faltblisters schematisch dargestellt. In der **Figur 2a** ist ein Faltblister mit mehreren mit einem Medikament gefüllten Taschen 2, mit einer vordersten, als nächste zu öffnenden Tasche 2" und mit einer geöffneten Tasche gezeigt. Die geöffnete Tasche befindet sich in einer Entnahmeposition, welche schematisch durch eine Inhalationskammer 7 angedeutet ist.

Der Blister wird zwischen zwei Transporträdern 8, einem oberen und einem unteren, geführt. Diese können mit einem Verriegelungsmechanismus ausgestattet sein, um den Blister an einer gewünschten Position, d.h. nach dem Durchgang der zu öffnenden Medikamententasche 2", festzuhalten. Es ist auch möglich nur ein unteres oder oberes Transportrad 8 vorzusehen, wobei vorzugsweise auf beiden Längsseiten des Blisterbandes je ein Transportrad angeordnet ist. Der Blister bzw. die zu öffnende Medikamententasche wird vorzugsweise genau an einer Stelle angehalten, derart dass sie nach einem Öffnen direkt in einer Entnahmeposition zu liegen kommt. Während die Transporträder in Bezug auf eine Transportrichtung des Blisters, hinter einer Tasche angeordnet sind, ist, wiederum in Transportrichtung gesehen, vor der zu öffnenden Tasche ein Öffnungsrad 9 angebracht. Mit Hilfe des Öffnungsrades, welches vorzugsweise ebenfalls mittels Stacheln in die Öffnungen des Blisters eingreift und beidseitig des Blisters angeordnet ist, kann das Folienband vorderseitig weitergezogen und die Tasche 2" geöffnet werden. Dabei wird das Öffnungsrad, welches ein Zahnrad sein kann, um die zum Öffnen der Tasche benötigte Distanz gedreht. Aufgrund dessen, dass auch das bzw. die Öffnungsräder seitlich zur Medikamententasche angeordnet sind, kann eine mit Medikament gefüllte Vertiefung zwischen den zwei seitlich angeordneten Öffnungsrädern vorbeigezogen werden. Die Öffnungsräder dienen dabei auch als Führung.

Das Öffnungsrad kann im wesentlichen als Transportelement ausgestaltet sein und um eine entsprechende Distanz zusammen mit und parallel zu einem vorderen Teil des Folienbandes in Transportrichtung bewegt werden und dabei eine Tasche öffnen.

Die Zugkraft zum Öffnen der Taschen geht vorzugsweise vom Öffnungsrad aus, kann aber auch von einer Aufwickelrolle 10 ausgehen, auf welches das verbrauchte und geöffnete Folienband aufrollt wird. Eine Aufwickelrolle 10 wäre mit einem entsprechenden Schlupf versehen, um unterschiedliche Durchmesser ausgleichen zu können. Geht eine Zugkraft vom einem Öffnungsrad aus, kann das verbrauchte Folienband beispielsweise auch einfach in ein dafür vorgesehenes Volumen in einer Ausgabevorrichtung gelangen oder aus dieser austreten, wo das Band abgetrennt und entfernt werden kann.

In den **Figuren 2b und 2c** ist die zu öffnende Tasche 2' in verschiedenen Transport- und Öffnungsstadien gezeigt. Dabei sind die Transport- und Öffnungsräder 8, 9 als Führungen für eine mit einer Vertiefung und Abdeckung versehene Tasche gestaltet. Aufgrund der unterstützenden Funktion des Öffnungsrades wird die Vertiefung in eine horizontale Lage gebracht und darin gehalten. Diese Massnahmen verhindern, dass während des Öffnens Pulver aus einer Tasche austreten kann, z.B. aufgrund einer Neigung des Folienblisters oder von allfälligen Kraftimpulsen, die während des Öffnens oder Transportierens auftreten könnten.

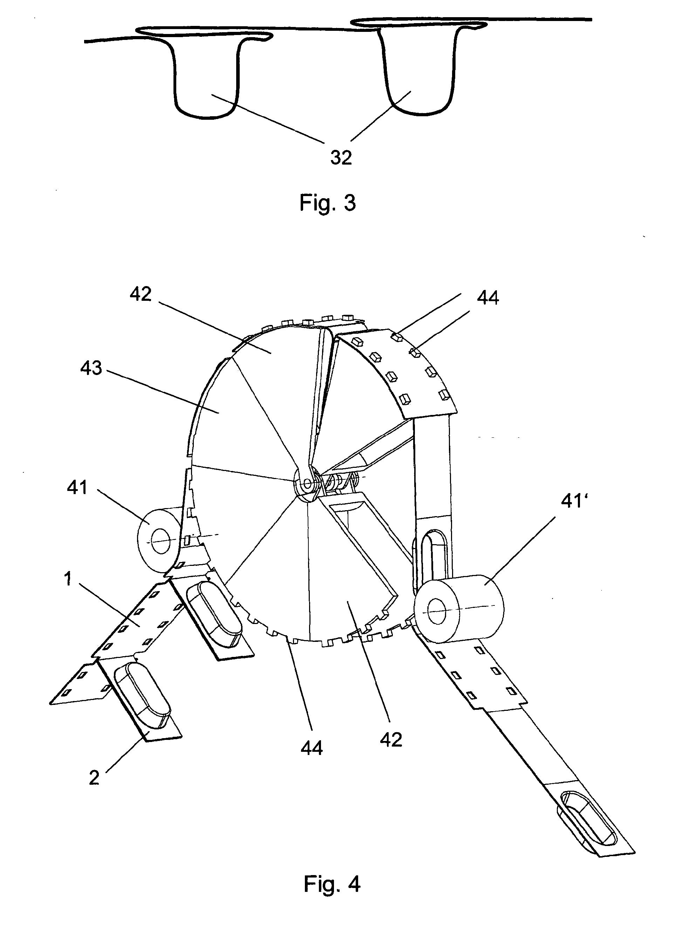

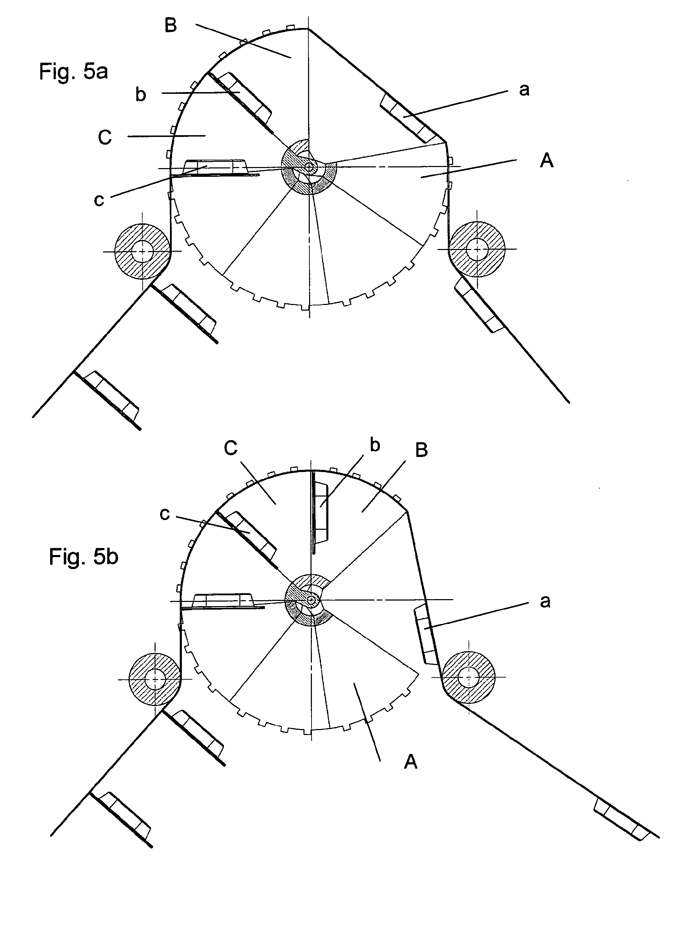

In einer bevorzugten Ausführungsform übernimmt ein Segmentrad das Transportieren und Öffnen des erfindungsgemässen Folienblisters, wie weiter unten in den Figuren 4 und 5a-d gezeigt.

In **Figur 3** ist eine weitere Gestaltung bzw. Herstellungsart eines Einfolien-Faltblisters gezeigt. Dabei wird mit einem Folienband eine Tasche 32 geformt. Die Tasche wird seitlich versiegelt (in der Figur nicht zu sehen), ein Medikament wird eingebracht und die Folie wird über die Einfüllöffnung der Tasche gelegt, wo sie ebenfalls angesiegelt wird und dabei die Tasche verschliesst. Schmalere Taschenbereiche und seitliche Öffnungen im Blister müssen nicht vorgängig im Folienband eingebracht werden, sondern können beispielsweise zeitgleich mit einer seitlichen Siegelung beziehungsweise mit einer oberen Siegelung der Taschen vorgenommen werden.

In **Figur 4,** sowie **Figur 5a-d** ist ein Segmentrad und ein damit verbundener Öffnungsvorgang eines erfindungsgemässen Faltblisters dargestellt.

Der Folienblister 1 ist wiederum aus einem Folienband gebildet, in dem eine Vielzahl von Medikamententaschen 2 geformt ist. Das Folienband weist mehrere regelmässig hintereinander angeordnete Öffnungen in den seitlichen Randbereichen des Folienbandes zwischen den Taschen auf. Die Folienbereiche, welche die Taschen bilden, sind schmaler und weisen keine Öffnungen für Transportstacheln auf. Die Länge der Bereiche zwischen den Taschen korrespondiert mit der Länge eines Kreisbogensegments des Umfangs des Segmentrades 43, welches Kreisbogensegment durch eine Stirnseite eines einzelnen Segments 42 gebildet wird. Die Form und Anzahl der im Folienband eingebrachten Öffnungen korrespondieren mit Vorsprüngen, die als Transportstacheln dienen, hier jeweils vier Vorsprünge, die in zwei Reihen angeordnet sind. Die Vorsprünge befinden sich an zwei parallel zueinander angeordneten Segmentseiten und weisen radial nach aussen. Der Faltblister wird über die ineinandergreifenden Öffnungen und Transportstacheln über einen Teil des Segmentrades auf und wieder abgerollt.

Die einzelnen Segmente weisen einen U-förmigen Querschnitt auf, derart, dass eine Medikamententasche im Hohlraum zwischen den Segmentseiten zu liegen kommt. Die Segmentseiten, und damit die Transportstachelreihen oder einzelne Transportstacheln, sind um mindestens eine Taschenbreite voneinander beabstandet. Vorzugsweise sind sie nur wenig mehr als eine Taschenbreite voneinander beabstandet, um ein möglichst reibungsfreies Transportieren eines Blisters zu ermöglichen, um eine gute Führung zu bilden und um möglichst Platz sparend angeordnet zu sein. Das Segmentrad ist aus mehreren, einzeln bewegbaren Segmenten, hier sechs Segmenten, gebildet, wobei alle Segmente zusammengeführt, keinen vollständigen Kreis bilden. Der freie Radbereich, der zwischen zwei Segmenten gebildet wird, wird wenn von einem einzelnen Segment überstrichen, zum Öffnen einer Medikamententasche verwendet. Er ist entsprechend auf die Länge bzw. die doppelte Länge einer Medikamententasche eingestellt. Blisterband und Segmente sind zudem so aufeinander abgestimmt, dass eine Medikamententasche genau zwischen zwei Segmenten zu liegen kommt. Durch voneinander Trennen der beiden Segmente wird dabei der Folienabschnitt, welcher eine Medikamententasche bildet, auseinandergezogen und gibt die Medikamentenkammer und das darin befindliche Medikament frei.

Unmittelbar vor und nach dem Segmentrad sind zwei Halterollen 41, 41' angebracht, welche den Faltblister über und an dem Segmentrad in Position halten.

Ein Öffnungsvorgang mittels des Segmentrades kann wie in den Figuren 5a-d gezeigt ablaufen. Der Faltblister wird über das Segmentrad geführt, wobei die Transportstacheln der einzelnen Segmente in die Öffnungen im Blister eingreifen. Durch eine Drehbewegung des Rades wird der Blister mit dem Rad transportiert. In **Figur 5a** ist der Blister in einer Entnahmeposition mit entleerter Medikamentenkammer a gezeigt. Die Entnahmeposition befindet sich in einem segmentfreien Bereich, wobei sämtliche Segmente aneinander anliegend weitertransportiert werden.

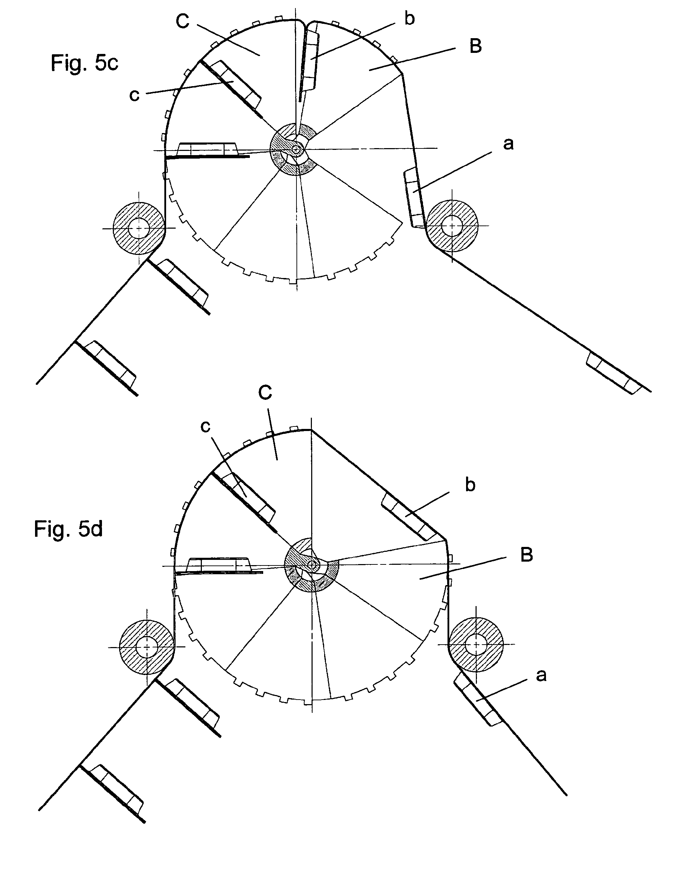

Die Drehbewegung des Segmentrades wird solange weitergeführt bis eine nächste Medikamententasche b in einer Öffnungsposition ist, wie in **Figur 5b** gezeigt. In diesem Punkt wird über einen Mechanismus, beispielsweise in der Nabe des Segmentrades, ein Arretiermechanismus ausgelöst, derart, dass das Blisterband vor der Öffnungsposition angehalten wird. Lediglich das nächste Segment B, auf das unmittelbar die als nächste zu öffnende Medikamententasche b folgt, wird in Transportrichtung weiterbewegt (**Figur 5c**). Aufgrund der auf das Folienband ausgeübte Kraft, öffnet sich die Tasche b und kommt, wie in **Figur 5d** gezeigt, offen und zur Entnahme bereit, in der Entnahmeposition zu liegen. Die Vorsprünge des Segments B halten die Folie am Segment B, während die Vorsprünge am Segment C, die Folie am Segment C festhalten. Durch die Bewegung des Segments B weiter in Transportrichtung wird die Folie solange gezogen, bis sich die Siegelung zwischen den Folienbereichen der Medikamententaschen voneinander lösen und sich die Tasche öffnet.

Das nächste Segment B schliesst nun an die bis zu diesem Punkt stillstehenden übrigen Segmente des Rades an und das Verfahren gemäss Figur 5a-d kann mit der übernächsten Medikamentenkammer c und dem übernächsten Segment C wiederholt werden. Während dem Öffnungsprozess wird die verbrauchte Kammer a über die Halterolle 41' weggeführt, z. B. auf eine Aufwickelrolle aufgerollt oder aus einer Medikamentenvorrichtung ausgeführt, wo das Folienstück mit der verbrauchten Kammer entfernt werden kann.

Der Transport des Segmentrades, inklusive dem Öffnungsmechanismus einer Medikamententasche kann mit einem Öffnungsmechanismus einer Medikamentenausgabevorrichtung, beispielsweise einem Öffnen einen Mundstücks eines Mehrdosispulverinhalators kombiniert werden.

Das Segmentrad und die beiden Halterollen sind ortsfest und um ihre jeweilige Rotationsachse rotierbar in einer Medikamentenausgabevorrichtung, wie beispielsweise einem Inhalator, angebracht.

## Patentansprüche

1. Medikamentenmagazin mit einer Mehrzahl von Medikamentendosen, wobei das Magazin aus einer einzigen Folienbahn gebildet ist, in welcher Taschen (2, 32) zur Aufnahme eines Medikaments geformt sind, wobei die Folienbahn für den Transport der Bahn auf mindestens einer Seite Öffnungen (4) aufweist für den Eingriff von Transportstacheln, **dadurch gekennzeichnet, dass** die Folienbahn im Bereich der Taschen eine gewisse Breite aufweist, welche kleiner ist als die Breite der Folie in übrigen Bereichen, und dass die Öffnungen in diesen breiteren Bereichen der Folienbahn angeordnet sind.

2. Medikamentenmagazin nach Anspruch 1, wobei sich die beiden Folienbreiten im wesentlichen um die Breite der Öffnungen (4) unterscheiden.

3. Medikamentenmagazin nach Anspruch 1 oder 2, wobei die Öffnungen (4) nur in Bereichen zwischen Taschen (2, 32) angeordnet sind.

4. Medikamentenmagazin gemäss einem der vorangehenden Ansprüche, wobei die Öffnungen (4) der Folienbahn für den Eingriff von Transportstacheln auch zum Öffnen der Taschen (2, 32) verwendbar sind.

5. Medikamentenmagazin gemäss einem der vorangehenden Ansprüche, wobei die Folienbahn auf beiden Längsseiten Öffnungen (4) aufweist.

6. Medikamentenmagazin gemäss einem der vorangehenden Ansprüche, wobei die Öffnungen (4) länglich sind und entsprechend ihrer Funktion unterschiedliche Längen aufweisen.

7. Medikamentenmagazin gemäss einem der vorhergehenden Ansprüche, wobei eine Tasche (2, 32) durch eine Vertiefung in der Folienbahn und eine im wesentlichen ebene Abdeckung gebildet ist.

8. Vorrichtung zum Öffnen eines Medikamentenmagazins gemäss einem der Ansprüche 1 bis 7, wobei sie mindestens einen bewegbaren Stachel zum Eingriff in mindestens eine dafür vorgesehene Öffnung (4) des Magazins aufweist, und dass sie Mittel für einen Transportschub eines Magazins und Mittel für einen Öffnungsschub eines Magazins zum Öffnen einer in einer Folienbahn eingebrachten Tasche (2, 32) aufweist, **dadurch gekennzeichnet, dass** der mindestens eine Stachel derart angeordnet ist, dass er in Bezug auf eine Transportrichtung seitlich und beabstandet zur Tasche in die Folienbahn eingreift.

9. Vorrichtung gemäss Anspruch 8, wobei der Stachel Teil eines Zahnrades ist.

10. Vorrichtung gemäss einem der Ansprüche 8 oder 9, wobei mindestens zwei Stacheln parallel zueinander und um mindestens eine Taschenbreite beabstandet voneinander angeordnet sind.

11. Vorrichtung gemäss einem der Ansprüche 8-10, wobei die Mittel für einen Transportschub eines Magazins und die Mittel für einen Öffnungsschub in einem diskontinuierlichen Stachelrad, z. B. einem Segmentrad (43), vereint sind.

12. Vorrichtung gemäss Anspruch 11, wobei das Segmentrad (43) mehrere Segmente (42) aufweist, und ein maximaler Abstand zwischen zwei benachbarten Segmenten im wesentlichen einer Bandlänge einer geöffneten Medikamententasche entspricht.

13. Verfahren zum Transportieren und Öffnen eines Medikamentenmagazins gemäss einem der Ansprüche 1 bis 7, wobei mindestens ein Stachel in eine dafür vorgesehene Öffnung (4) des mindestens eine Medikamententasche (2, 32) aufweisenden Medikamentenmagazins eingreift und durch eine Transportbewegung das Magazin in eine Öffnungsposition bringt, und dass dann das Magazin vor der mindestens einen Medikamententasche im Bereich der Öffnungsposition festgehalten und nach dieser Medikamententasche mittels dem mindestes einen Stachel weitertransportiert wird, derart dass die Medikamententasche aufgezogen und **dadurch** das in der Medikamententasche befindliche Medikament frei gegeben wird.

14. Verfahren gemäss Anspruch 13, wobei eine Medikamententasche (2, 32) durch mindestens zwei in das Medikamentenmagazin eingreifende und sich relativ voneinander wegbewegende Stacheln geöffnet wird.

15. Verfahren gemäss Anspruch 13 oder 14, wobei eine zwischen zwei Segmenten (42) eines Segmentrades (43) angeordnete Medikamententasche (2, 32) durch Auseinander bewegen der beiden Segmente geöffnet wird.

16. Mehrdosispulverinhalator aufweisend ein Medikamentenmagazin gemäss einem der Ansprüche 1 bis 7 und/oder einer Vorrichtung gemäss einem der Ansprüche 8 bis 12.

17. Mehrdosispulverinhalator gemäss Anspruch 16 aufweisend 60 Medikamenteneinzeldosen.

18. Mehrdosispulverinhalator gemäss Anspruch 16 oder 17 zur Applikation eines Arzneimittels, das einen Wirkstoff oder eine Kombination der Wirkstoffe enthält ausgewählt aus der Gruppe Betamimetika, Anticholinergika, Steroide, Antiallergika, Derivate von Mutterkornalkaloiden, Triptane, CGRP-Antagonisten, der Phosphodiesterase -V-Inhibitoren, Phosphodiesterase-IV-Inhibitoren, LTD4-Antagonisten, EGFR-Kinase-Hemmer.
